# EUROPEAN PATENT SPECIFICATION

(11) **EP 2 964 282 B1**
(45) Date of publication and mention of the grant of the patent: **15.05.2019**
(21) Application number: 14711372.4
(22) Date of filing: 28.02.2014
(51) Int. Cl.: A61L 31/14, A61L 31/16

(54) **IMPLANTABLE MEDICAL DEVICE COMPRISING A MACROCYCLIC TRIENE LACTONE DRUG AND MINIMAL ANTIOXIDANT STABILIZER AND METHOD OF FABRICATION**
IMPLANTIERBARE MEDIZINISCHE VORRICHTUNG MIT EINEM MAKROCYCLISCHEN LACTON-TRIEN-LACTON-ARZNEIMITTEL UND EINEM MINIMAL ANTIOXIDIERENDEN STABILISATOR SOWIE VERFAHREN ZUR HERSTELLUNG
DISPOSITIF MÉDICAL IMPLANTABLE COMPRENANT UN MÉDICAMENT TRIÈNE LACTONE MACROCYCLIQUE ET UN STABILISANT ANTI-OXYDANT MINIMAL ET PROCÉDÉ DE FABRICATION

(30) Priority: 07.03.2013 US 201313788584
(43) Date of publication of application: 13.01.2016
(73) Proprietor: Abbott Cardiovascular Systems Inc., Santa Clara, California 95054-2809 (US)
(72) Inventor: PACETTI, Stephen D., San Jose, California 95130 (US); GUO, Sherry Xuan, San Jose, California 95132 (US)
(74) Representative: ZBM Patents - Zea, Barlocci & Markvardsen
(86) International application number: PCT/US2014/019317
(87) International publication number: WO 2014/137787

(56) References cited:
- EP-A2- 1 586 338
- WO-A1-2012/017449
- WO-A1-2012/162007

## Description

### FIELD

This invention relates to an implantable medical device (IMD) comprising an oxygen-sensitive macrocyclic triene lactone drug, which is protected from oxidative degradation by addition of an antioxidant stabilizer, the amount of which is reduced from an initial effective concentration to a minimal or non-detect amount by the time the IMD has been fabricated, sterilized and packaged under an inert atmosphere in a light-tight container. A method of achieving the minimal antioxidant final product is also presented.

### BACKGROUND

Until the mid-1980s, the accepted treatment for coronary atherosclerosis, i.e., narrowing of the coronary artery(ies) was coronary by-pass surgery. While being quite effective and having evolved to a relatively high degree of safety for an invasive procedure, by-pass surgery still involves potentially serious complications and generally results in an extended recovery period.

With the advent of percutaneous transluminal coronary angioplasty (PTCA) in 1977, the scene changed dramatically. Using catheter techniques originally developed for heart exploration, inflatable balloons were deployed to re-open occluded regions in arteries. The procedure was relatively non-invasive, took a short time compared to by-pass surgery and recovery time was minimal. However, PTCA brought with it its own problems including vasospasm, elastic recoil of the stretched arterial wall and restenosis, the re-clogging of the treated artery due to neointimal hyperplasia in the vicinity of the procedure, any of which could undo much of what had been accomplished.

The next improvement, advanced in the mid-1980s, was the use of a stent to maintain a luminal diameter that had been re-established using PTCA. This for all intents and purposes put an end to vasospasm and elastic recoil but did not resolve the issue of restenosis. That is, prior to the introduction of stents, restenosis occurred in about 30 to 50% of patients undergoing PTCA. Stenting reduced this to about 15 to 30%, a substantial improvement but still more than desirable.

In 2003, the drug-eluting stent (DES) was introduced. The drugs initially used with DESs were cytostatic compounds, that is, compounds that curtailed the proliferation of cells that fostered restenosis. With DESs, the occurrence of restenosis was reduced to about 5 to 7%, a relatively acceptable figure. However, the use of DESs engendered yet another complication, late stent thrombosis, the forming of blood clots at some time after the stent was in place. It was hypothesized that the formation of blood clots was most likely due to delayed healing, a side-effect of the use of cytostatic drugs. Thus, other types of drugs were sought to reduce the incidence of late stent thrombosis as well as other complications related to the use of cytostatic agents. A promising solution was found in the anti-proliferative family of compounds, in particular macrocyclic triene lactones, such as rapamycin, which appeared surprisingly effective. DESs comprising members of the rapamycin family of compounds were extensively studied and several have become commercial products. It was found, however, that, due at least in part to the fact that there are three conjugated double bonds in macrocyclic triene lactone family of compounds, the entire genus is sensitive to oxidative and free radical induced degradation. That is, oxygen in and around a DES containing a macrocyclic triene lactone fosters the formation of radical species that in turn initiate auto-oxidation of the triene moiety. The response to this negative property of the compounds was obvious to those skilled in the art: protect the macrocyclic triene lactone by including a pharmaceutically acceptable antioxidant with the drug both as an isolated solid as synthesized and purified and in a drug reservoir layer containing the macrocyclic triene lactone on a DES.

WO2012/162007 discloses a medical device comprising: an expandable member having a first diameter for insertion into a vessel and a second diameter for making contact with the vessel walls; and a non-aqueous formulation of a rapamycin, including synthetic and semi synthetic analogs thereof, affixed to and dried onto at least a portion of the surface of the expandable member, the dried, non-aqueous liquid formulation comprising a rapamycin, in a therapeutic dosage in the range of up to ten micrograms per square millimeter of expandable member surface area, an antioxidant in an amount of up to 5 percent by weight, a film forming agent in a pharmaceutically acceptable range of between 0.05 percent to about 20 percent by weight, and substantially no volatile, non-aqueous solvent.

WO2012/017449A1 discloses the synthesis of a rapamycin derivative. It also discloses that the rapamycin compound can be stabilized by addition of an antioxidant for enhancing storage stability.

EP1586338A2 discloses the use of antioxidants to prevent oxidation and reduce drug degradation in drug eluting medical devices.

The problem is that many antioxidants including those suitable for use on DESs are not particularly salutary to patients. This, together with the fact that, once fabricated and packaged in an essentially oxygen-free atmosphere protected from light, macrocyclic triene lactones are actually reasonably stable suggests that it would be beneficial to have an antioxidant present during the fabrication of a macrocyclic triene lactone-containing DES but have a little as possible remaining once the DES is mounted on a carrier vehicle, sterilized and packaged in a light-tight, inert atmosphere container or once the DES has been implanted in a patient. The present invention provides an antioxidant-stabilized macrocyclic triene lactone DES with minimal antioxidant at the point of packaging and a method of achieving the same.

### SUMMARY

Thus, it is disclosed an implantable medical device comprising a drug reservoir layer comprising a macrocyclic triene lactone drug and about 0.001% to about 0.01% by weight, based on the weight of macrocyclic triene lactone drug present, of a pharmaceutically acceptable antioxidant stabilizer.

In an aspect of this invention, the macrocyclic triene lactone drug is an amorphous solid.

In an aspect of this invention, the macrocyclic triene lactone drug is everolimus.

This invention relates to a method of fabricating an implantable medical device comprising a drug reservoir layer comprising a macrocyclic triene lactone drug and less than 0.01% by weight, based on the weight of macrocyclic triene lactone present, of a pharmaceutically acceptable antioxidant stabilizer, the method comprising,
providing an implantable medical device wherein:
The implantable medical device may be a device body or it may be a device body that has already been coated with one or more layers of material(s);
providing an essentially pure macrocyclic triene lactone drug;
providing a pharmaceutically acceptable antioxidant stabilizer in an amount of 0.02% to 0.1% by weight based on the weight of the macrocyclic triene lactone drug to be disposed on the device body;
combining the macrocyclic triene lactone drug and pharmaceutically acceptable antioxidant stabilizer;
dissolving or dispersing the combined macrocyclic triene lactone drug and pharmaceutically acceptable antioxidant stabilizer in a coating solvent; spray-coating the drug/stabilizer-containing solvent onto the implantable medical device;
drying the spray-coated implantable medical device at an elevated temperature that has been determined to not detrimentally affect the macrocyclic triene lactone drug; mounting the dried spray-coated implantable medical device on a carrier vehicle; and sterilizing the mounted implantable medical device/carrier vehicle.

In an aspect of this invention, in the above method, a matrix polymer is added to the macrocyclic triene lactone and antioxidant stabilizer in the dissolution or dispersion step.

In this invention, in the above method, subsequent to mounting and prior to sterilization, the mounted implantable medical device/carrier vehicle is packaged in a gas permeable container.

In this invention, in the above method, sterilization comprises ethylene oxide.

In an aspect of this invention, in the above method, the sterilized implantable medical device/carrier vehicle/gas permeable container is further packaged in a light-tight container under an inert atmosphere.

In an aspect of this invention, in the above method, combining the macrocyclic triene lactone with the pharmaceutically acceptable antioxidant stabilizer comprises mechanically reducing the particle size of each substance in the solid state to form micro-scale or nano-scale powders, either separately, after which the powders are mixed together to form a substantially homogeneous mixed powder or mixing the solid substances together before mechanically reducing particle size.

In an aspect of this invention, in the above method, combining the macrocyclic triene lactone with the pharmaceutically acceptable antioxidant stabilizer comprises dissolving both in a water miscible solvent and then adding the solution to a volume of water to co-precipitate the macrocyclic triene lactone and antioxidant stabilizer, the co-precipitant then being used in the dissolution/dispersion step.

In an aspect of this invention, in the above method, the implantable medical device is a stent.

In an aspect of this invention, in the above method, the macrocyclic triene lactone drug is selected from the group consisting of rapamycin, a 40-O-substituted rapamycin, a 16-O-substituted rapamycin, a rapamycin derivative, 32-deoxorapamycin, zotarolimus, everolimus, temsirolimus, deforolimus, ridaforolimus, merilimus, biolimus, umirolimus, novolimus and myolimus.

In an aspect of this invention, in the above method, the pharmaceutically acceptable antioxidant stabilizer is selected from the group consisting of a butylated phenol, butylated hydroxytoluene (BHT), butylated hydroxyanisole, t-butylhydroquinone, quinone, an alkyl gallate, methyl gallate, ethyl gallate, propyl gallate, octyl gallate, docecyl gallate, resveratrol, cysteine, n-acetylcysteine, bucillamine, glutathione, 7-hydroxyethylrutoside, carvedilol, vitamin C, ascorbyl palmitate, fumaric acid, a tocopherol, α-tocopherol, D,L-α-tocopherol, α-tocopherol acetate, a tocotrienol, vitamin E, lycopene, a flavonoid, a carotenoid and carotene.

In an aspect of this invention, in the above method, the pharmaceutically acceptable antioxidant stabilizer is BHT.

In an aspect of this invention, in the above method, combining the macrocyclic triene lactone drug with the BHT comprises mixing the macrocyclic triene lactone solid, which has been ground to a micro- or nano-scale powder, and the BHT in a vessel under reduced pressure and heating the mixture to a temperature above the sublimation temperature of the BHT at that reduced pressure.

In as aspect of this invention, in the above method, the macrocyclic triene lactone is selected from the group consisting of everolimus and zotarolimus.

### DETAILED DESCRIPTION

### Brief description of the figures

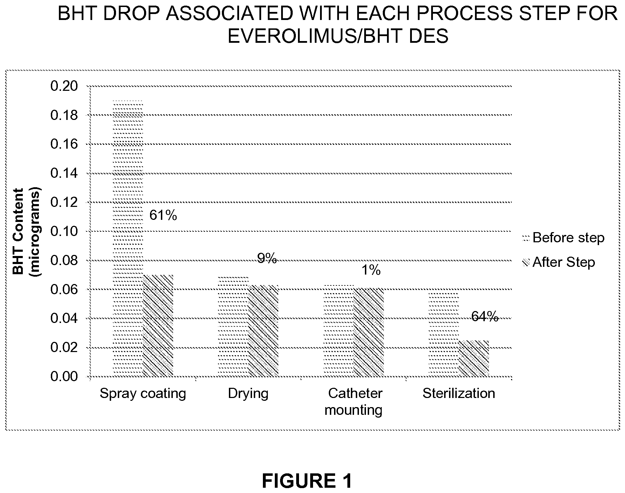

**Figure 1** is a graph showing the decrease in BHT content on a stent due to each step in the manufacturing process in which a macrocyclic triene lactone and BHT are present.

### Discussion

Use of the singular herein includes the plural and *vice versa* unless expressly otherwise stated. That is, "a" and "the" refer to one or more of whatever the word modifies. For example, "a pharmaceutically acceptable antioxidant" includes one such oxidant, two such oxidants or, under the right circumstances, an even greater number of antioxidants. By the same token, words such as, without limitation, "coatings" and "layers" refer to one coating or layer as well as to a plurality of coatings or layers unless, again, it is expressly stated or obvious from the context that such is not intended.

As used herein, words of approximation such as, without limitation, "about" "substantially," "essentially" and "approximately" mean that the feature so modified need not be exactly that which is expressly described but may vary from that written description to some extent. The extent to which the description may vary will depend on how great a change can be instituted and have one of ordinary skill in the art recognize the modified feature as still having the required characteristics and capabilities of the unmodified feature. In general, but subject to the preceding discussion, a numerical value herein that is modified by a word of approximation such as "about" may vary from the stated value by ±15%.

A "macrocyclic triene lactone" will often be abbreviated herein as an "MTL."

As used herein, an "implantable medical device" refers to any type of appliance that is totally or partly introduced, surgically or medically, into a patient's body or by medical intervention into a natural orifice, and which is intended to remain there after the procedure. The duration of implantation may be essentially permanent, i.e., intended to remain in place for the remaining lifespan of the patient; until the device is physically removed; or until the device biodegrades usually as the intentional use of a biodegradable substance for the fabrication of the device such that the device degrades over a predetermined time-span. Examples of implantable medical devices include, without limitation, implantable cardiac pacemakers and defibrillators; leads and electrodes for the preceding; implantable organ stimulators such as nerve, bladder, sphincter and diaphragm stimulators, cochlear implants; prostheses, vascular grafts, self-expandable stents, balloon-expandable stents, stent-grafts, grafts, artificial heart valves and cerebrospinal fluid shunts.

A presently preferred implantable medical device for the purposes of this invention is a stent.

A stent refers generally to a device used to hold tissue in place in a patient's body. Particularly useful stents, however, are those used for the maintenance of the patency of a vessel in a patient's body when the vessel is narrowed or closed due to diseases or disorders including, without limitation, tumors (in, for example, bile ducts, the esophagus, the trachea/bronchi, etc.), benign pancreatic disease, coronary artery disease, carotid artery disease and peripheral arterial disease such as atherosclerosis, restenosis and vulnerable plaque. Vulnerable plaque (VP) refers to a fatty build-up in an arterial wall thought to be caused by inflammation and atherosclerosis. The VP is covered by a thin fibrous cap that can rupture leading to blood clot formation. A stent can be used to strengthen the wall of the vessel in the vicinity of the VP and act as a shield against such rupture. A stent can be used, without limitation, in the neurological, carotid, coronary, pulmonary, renal, iliac, femoral, popliteal and tibial arteries as well as in biliary applications and other peripheral vasculatures. A stent can be used in the treatment or prevention of disorders such as, without limitation, thrombosis, restenosis, hemorrhage, vascular dissection or perforation, vascular aneurysm, chronic total occlusion, claudication, anastomotic proliferation, bile duct obstruction and ureter obstruction.

In addition to the above uses, stents may also be employed for the localized delivery of therapeutic agents to specific treatment sites in a patient's body. In fact, therapeutic agent delivery may be the sole purpose of the stent or the stent may be primarily intended for another use such as those discussed above with drug delivery providing an ancillary benefit. The DES or "drug eluting stent" of this invention is a non-limiting example of an implantable medical device. The primary purpose of a DES is to maintain the patency of a vascular lumen, while the drug on the stent serves to mitigate medical conditions ancillary to the implantation of the stent.

A stent used for patency maintenance is usually delivered to the target site in a compressed state and then expanded to fit the vessel into which it has been inserted. Once at a target location, a stent may be self-expandable or balloon expandable. In any event, due to the expansion of the stent, any coating thereon must be flexible and capable of elongation.

As used herein, "device body" refers to a fully formed implantable medical with an outer surface to which no coating or layer of material different from that of which the device itself is manufactured has been applied. A common example of a device body is a bare metal stent (BMS), which, as the name implies, is a fully-formed, usable stent that has not been coated with a layer of any material different from the metal of which it is made on any surface that is in contact with bodily tissue or fluids. Of course, device body refers not only to BMSs but to any uncoated device regardless of what it is made. Device bodies comprised of bioresorbable polymers and corrodible metals are also known.

Implantable medical devices made of virtually any material, i.e., materials presently known to be useful for the manufacture of implantable medical devices and materials that may be found to be so in the future, may be used in the method of this invention. For example, without limitation, an implantable medical device useful with this invention may be made of one or more biocompatible metals or alloys thereof including, but not limited to, cobalt-chromium alloy (ELGILOY, L-605), cobalt-nickel alloy (MP-35N), 316L stainless steel, high nitrogen stainless steel, e.g., BIODUR 108, nickel-titanium alloy (NITINOL), iron-platinum-chromium alloy, tantalum, platinum, platinum-iridium alloy, iron-plantinum-chromium alloy, gold and combinations thereof.

Implantable medical devices may also be made of polymers that are biocompatible and biostable or biodegradable, the latter term including bioabsorbable, bioresorbable and/or bioerodable.

As used herein, a "biocompatible" polymer refers to a polymer that both in its intact as synthesized state and in its decomposed state, i.e., its degradation products, is not, or at least is minimally toxic to living tissue; does not, or at least minimally and reparably injures living tissue; and/or does not, or at least minimally and/or controllably causes an immunological reaction in living tissue. Biocompatible polymers of this invention may be biostable or biodegradable where "biodegradable" simply means that the polymer will be decomposed over time when exposed to a physiological environs, i.e. to the conditions present in a patient's body such as pH, the presence of enzymes, body temperature, etc. "Biostable," on the other hand, refers to a polymer that does not significantly break down under physiological conditions for essentially the entire duration of its residency in a patient's body.

Examples of biocompatible, relatively biostable polymers that may be used with an implantable medical device of this invention include, without limitation, polyacrylates, polymethacryates, polyureas, polyurethanes, polyolefins, polyvinylhalides, polyvinylidenehalides, polyvinylethers, polyvinylaromatics, polyvinylesters, polyacrylonitriles, polysiloxanes, alkyd resins and epoxy resins.

Biocompatible, biodegradable polymers include naturally-occurring polymers such as, without limitation, collagen, chitosan, alginate, fibrin, fibrinogen, cellulosics, starches, dextran, dextrin, hyaluronic acid, heparin, glycosaminoglycans, polysaccharides and elastin.

One or more synthetic or semi-synthetic biocompatible, biodegradable polymers may also be used to fabricate an implantable medical device body of this invention. As used herein, a synthetic polymer refers to one that is created wholly in the laboratory while a semi-synthetic polymer refers to a naturally-occurring polymer that has been chemically modified in the laboratory. Examples of synthetic polymers include, without limitation, polyphosphazines, polyphosphoesters, polyphosphoester urethane, polyhydroxyacids, polyhydroxyalkanoates, polyanhydrides, polyesters, polyorthoesters, polyamino acids, polyoxymethylenes, poly(ester-amides) and polyimides.

Other biocompatible polymers that may be used to fabricate the device to be coated with a macrocyclic triene lactone drug/antioxidant stabilizer drug reservoir layer of this invention include, without limitations, polyesters, polyhydroxyalkanoates (PHAs), poly(ester amides) that may optionally contain alkyl, amino acid, PEG and/or alcohol groups, polycaprolactone, poly(L-lactide), poly(D,L-lactide), poly(D,L-lactide-co-PEG) block copolymers, poly(D,L-lactide-co-trimethylene carbonate), polyglycolide, poly(lactide-co-glycolide), polydioxanone (PDS), polyorthoester, polyanhydride, poly(glycolic acid-co-trimethylene carbonate), polyphosphoester, polyphosphoester urethane, poly(amino acids), polycyanoacrylates, poly(trimethylene carbonate), poly(iminocarbonate), polycarbonates, polyurethanes, copoly(ether-esters) (e.g. PEO/PLA), polyalkylene oxalates, polyphosphazenes, PHA-PEG, and combinations thereof. The PHA may include poly(α-hydroxyacids), poly(β-hydroxyacid) such as poly(3-hydroxybutyrate) (PHB), poly(3-hydroxybutyrate-co-valerate) (PHBV), poly(3-hydroxyproprionate) (PHP), poly(3-hydroxyhexanoate) (PHH), or poly(4-hydroxyacid) such as poly poly(4-hydroxybutyrate), poly(4-hydroxyvalerate), poly(4-hydroxyhexanoate), poly(hydroxyvalerate), poly(tyrosine carbonates), poly(tyrosine arylates), poly(ester amide), polyhydroxyalkanoates (PHA), poly(3-hydroxyalkanoates) such as poly(3-hydroxypropanoate), poly(3-hydroxybutyrate), poly(3-hydroxyvalerate), poly(3-hydroxyhexanoate), poly(3-hydroxyheptanoate) and poly(3-hydroxyoctanoate), poly(4-hydroxyalkanaote) such as poly(4-hydroxybutyrate), poly(4-hydroxyvalerate), poly(4-hydroxyhexanote), poly(4-hydroxyheptanoate), poly(4-hydroxyoctanoate) and copolymers including any of the 3-hydroxyalkanoate or 4-hydroxyalkanoate monomers described herein or blends thereof, poly(D,L-lactide), poly(L-lactide), polyglycolide, poly(D,L-lactide-co-glycolide), poly(L-lactide-co-glycolide), polycaprolactone, poly(lactide-co-caprolactone), poly(glycolide-co-caprolactone), poly(dioxanone), poly(ortho esters), poly(anhydrides), poly(tyrosine carbonates) and derivatives thereof, poly(tyrosine ester) and derivatives thereof, poly(imino carbonates), poly(glycolic acid-co-trimethylene carbonate), polyphosphoester, polyphosphoester urethane, poly(amino acids), polycyanoacrylates, poly(trimethylene carbonate), poly(iminocarbonate), polyphosphazenes, silicones, polyesters, polyolefins, polyisobutylene and ethylene-alphaolefin copolymers, acrylic polymers and copolymers, vinyl halide polymers and copolymers, such as polyvinyl chloride, polyvinyl ethers, such as polyvinyl methyl ether, polyvinylidene halides, such as polyvinylidene chloride, polyacrylonitrile, polyvinyl ketones, polyvinyl aromatics, such as polystyrene, polyvinyl esters, such as polyvinyl acetate, copolymers of vinyl monomers with each other and olefins, such as ethylene-methyl methacrylate copolymers, acrylonitrile-styrene copolymers, ABS resins, and ethylene-vinyl acetate copolymers, polyamides, such as Nylon 66 and polycaprolactam, alkyd resins, polycarbonates, polyoxymethylenes, polyimides, polyethers, poly(glyceryl sebacate), poly(propylene fumarate), poly(n-butyl methacrylate), poly(sec-butyl methacrylate), poly(isobutyl methacrylate), poly(tert-butyl methacrylate), poly(n-propyl methacrylate), poly(isopropyl methacrylate), poly(ethyl methacrylate), poly(methyl methacrylate), epoxy resins, polyurethanes, rayon, rayon-triacetate, cellulose acetate, cellulose butyrate, cellulose acetate butyrate, cellophane, cellulose nitrate, cellulose propionate, cellulose ethers, carboxymethyl cellulose, polyethers such as poly(ethylene glycol) (PEG), copoly(ether-esters) (e.g. poly(ethylene oxide-co-lactic acid) (PEO/PLA)), polyalkylene oxides such as poly(ethylene oxide), poly(propylene oxide), poly(ether ester), polyalkylene oxalates, phosphoryl choline containing polymer, choline, poly(aspirin), polymers and co-polymers of hydroxyl bearing monomers such as 2-hydroxyethyl methacrylate (HEMA), hydroxypropyl methacrylate (HPMA), hydroxypropylmethacrylamide, PEG acrylate (PEGA), PEG methacrylate, methacrylate polymers containing 2-methacryloyloxyethyl- phosphorylcholine (MPC) and n-vinyl pyrrolidone (VP), carboxylic acid bearing monomers such as methacrylic acid (MA), acrylic acid (AA), alkoxymethacrylate, alkoxyacrylate, and 3-trimethylsilylpropyl methacrylate (TMSPMA), poly(styrene-isoprene-styrene)-PEG (SIS-PEG), polystyrene-PEG, polyisobutylene-PEG, polycaprolactone-PEG (PCL-PEG), PLA-PEG, poly(methyl methacrylate)-PEG (PMMA-PEG), polydimethylsiloxane-co-PEG (PDMS-PEG), poly(vinylidene fluoride)-PEG (PVDF-PEG), PLURONIC™ surfactants (polypropylene oxide-co-polyethylene glycol), poly(tetramethylene glycol), hydroxy functional poly(vinyl pyrrolidone), biomolecules such as collagen, chitosan, alginate, fibrin, fibrinogen, cellulose, starch, dextran, dextrin, hyaluronic acid, fragments and derivatives of hyaluronic acid, heparin, fragments and derivatives of heparin, glycosamino glycan (GAG), GAG derivatives, polysaccharide, elastin, elastin protein mimetics, or combinations thereof. Some examples of elastin protein mimetics include (LGGVG)ₙ, (VPGVG)ₙ, Val-Pro-Gly-Val-Gly, or synthetic biomimetic poly(L-glytanmate)-b-poly(2-acryloyloxyethyllactoside)-b-poly(I-glutamate) triblock copolymer.

In some embodiments of the current invention the polymer used with the device and in the method of this invention can be poly(ethylene-co-vinyl alcohol), poly(methoxyethyl methacrylate), poly(dihydroxylpropyl methacrylate), polymethacrylamide, aliphatic polyurethane, aromatic polyurethane, nitrocellulose, poly(ester amide benzyl), co-poly-{[N,N'-sebacoyl-bis-(L-leucine)-1,6-hexylene diester]_{0.75}-[N,N'-sebacoyl-L-lysine benzyl ester]_{0.25}} (PEA-Bz), co-poly-{[N,N'-sebacoyl-bis-(L-leucine)-1,6-hexylene diester]_{0.75}-[N,N'-sebacoyl-L-lysine-4-amino-TEMPO amide]_{0.25}} (PEA-TEMPO), aliphatic polyester, aromatic polyester, fluorinated polymers such as poly(vinylidene fluoride-co-hexafluoropropylene), poly(vinylidene fluoride) (PVDF), poly(vinylidene fluoride-co-hexafluoropropylene-co-tetrafluoroethylene), and Teflon™ (polytetrafluoroethylene), a biopolymer such as elastin mimetic protein polymer, star or hyper-branched SIBS (styrene-block-isobutylene-block-styrene), or combinations thereof. In some embodiments, where the polymer is a copolymer, it can be a block copolymer that can be, e.g., di-, tri-, tetra-, or oligo block copolymers or a random copolymer. In some embodiments, the polymer can also be branched polymers such as star polymers.

Presently preferred polymers for use with this invention include polyesters such as, without limitation, poly(L-lactide), poly(D-lactide), poly(D,L-lactide), poly(meso-lactide), poly(L-lactide-co-glycolide), poly(D-lactide-co-glycolide), poly (D,L-lactide-co-glycolide), poly(meso-lactide-co-glycolide), poly(caprolactone), poly(glycolide-co-caprolactone), poly(D,L-lactide-co-caprolactone), poly(hydroxyvalerate), poly(hydroxybutyrate), poly(ethylene glycol-co-butylene terephthalate).

Other presently preferred polymers of this invention are fluoropolymers such as poly(vinylidene fluoride-co-hexafluoropropylene). When used, the poly(vinylidene fluoride-co-hexafluoropropylene) preferable at present has a constitutional unit weight-to-weight (wt/wt) ratio of about 85:15. "Constitutional unit" refers to the composition of a monomer as it appears in a polymer. For example, without limitation, the constitutional unit of the monomer acrylic acid, CH₂=CHC(O)OH, is-CH₂-CH(C(O)O)-. The average molecular weight of the presently preferred poly(vinylidene fluoride-co-hexafluoropropylene) polymer is from about 50,000 to about 500,000 Daltons. Further, it is presently preferred that the poly(vinylidene fluoride-co-hexafluoropropylene) polymer used to form a drug reservoir layer herein be semicrystalline. The presently preferred coating thickness of the poly(vinylidene fluoride-co-hexafluoropropylene) drug reservoir layer is from about 1 um to about 20 um.

Blends and copolymers of the above polymers may also be used and are within the scope of this invention. Based on the disclosures herein, those skilled in the art will recognize those implantable medical devices and those materials from which they may be fabricated that will be useful with the coatings of this invention.

As used herein, a "primer layer" refers to a coating consisting of a polymer or blend of polymers that exhibit good adhesion characteristics with regard to the material of which the device body is manufactured and good adhesion characteristic with regard to whatever material is to be coated on the device body. Thus, a primer layer serves as an intermediary layer between a device body and materials to be affixed to the device body and is, therefore, applied directly to the device body. Examples without limitation, of primers include acrylate and methacrylate polymers with poly(n-butyl methacrylate) being a presently preferred primer. Some additional examples of primers include, but are not limited to, poly(ethylene-co-vinyl alcohol), poly(vinyl acetate-co-vinyl alcohol), poly(methacrylates), poly(acrylates), polyethyleneamine, polyallylamine, chitosan, poly(ethylene-co-vinyl acetate), and parylene-C.

As used herein, "drug reservoir layer" refers either to a layer of therapeutic agent applied neat to a device body, which may already have a primer layer, or dissolved or dispersed in a polymer matrix, which is then applied to the IMD. A polymeric drug reservoir matrix is designed such that, by one mechanism or another, e.g., without limitation, by elution or as the result of biodegradation of the polymer, the therapeutic substance is released from the layer into the surrounding environment. A drug reservoir layer may also act as release rate-controlling layer.

In addition to an optional primer layer and a drug reservoir layer, an implantable medical device of this invention may comprise a topcoat layer. As used herein, a "topcoat layer" refers to a polymeric layer that is disposed over an implantable medical device of this invention such that it comprises the outermost layer of polymer on the device, that is, it is the layer that is in direct contact with the environment in which the device implanted. A topcoat layer may be biostable or biodegradable. Biodegradation may occur relatively slowly if the layer also serves as a rate controlling layer for the release of the macrocyclic triene lactone drug from the device, or biodegradation may occur rapidly if the topcoat layer serves only as a protective layer for the layers underneath. A topcoat layer may also serve as a compatibility-inducing layer that renders the device more inert with regard to reaction with foreign body-eliminating mechanisms with the body.

As use herein, a material that is described as a layer "disposed over" a particular substrate be it a device body or another layer, refers to a coating of the material applied directly to the exposed surface of the indicated substrate. By "exposed surface" is meant any surface regardless of its physical location with respect to the configuration of the device that, in use, would be in contact with bodily tissues or fluids. "Disposed over" may, however, also refer to the application of the layer onto an intervening layer that has been applied to a stent body, wherein the layer is applied in such a manner that, were the intervening layer not present, the layer would be applied to the exposed surface of the indicated substrate. An example of an intervening layer is a primer layer.

As used herein, the terms "drug," "therapeutic agent," "active agent" and the like are interchangeable and refer to substances that have been approved by the Food and Drug Administration (FDA), overseas regulatory agencies, notified bodies or the USDA for use in treatment of diseases and disorders in any animal species, but in particular human beings. In general, a drug, a therapeutic agent or an active agent refers to any substance that, when administered in a therapeutically effective amount to a patient suffering from a disease, has a therapeutic beneficial effect on the health and well-being of the patient. A therapeutic beneficial effect on the health and well-being of a patient includes, but it not limited to: (1) curing the disease; (2) slowing the progress of the disease; (3) causing the disease to retrogress; or, (4) alleviating one or more symptoms of the disease. As used herein, a therapeutic agent also includes any substance that when administered to a patient, known or suspected of being particularly susceptible to a disease, in a prophylactically effective amount, has a prophylactic beneficial effect on the health and well-being of the patient. A prophylactic beneficial effect on the health and well-being of a patient includes, but is not limited to: (1) preventing or delaying on-set of the disease in the first place; (2) maintaining a disease at a retrogressed level once such level has been achieved by a therapeutically effective amount of a substance, which may be the same as or different from the substance used in a prophylactically effective amount; or, (3) preventing or delaying recurrence of the disease after a course of treatment with a therapeutically effective amount of a substance, which may be the same as or different from the substance used in a prophylactically effective amount, has concluded.

A "therapeutically effective amount" refers to that amount of a therapeutic agent that will have a beneficial effect, which may be curative or palliative, on the health and well-being of the patient with regard to the disease or disorder with which the patient is known or suspected to be afflicted. A therapeutically effective amount may be administered as a single bolus, as intermittent bolus charges, as short, medium or long term sustained release formulations or as any combination of these. As used herein, short-term sustained release refers to the administration of a therapeutically effective amount of a therapeutic agent over a period from about several hours to about 3 days. Medium-term sustained release refers to administration of a therapeutically effective amount of a therapeutic agent over a period from about 3 day to about 14 days and long-term refers to the delivery of a therapeutically effective amount over any period in excess of about 14 days. Any reference a therapeutic agent relating to its presence on an implantable medical device or its use in a method of this invention is to be understood as referring to a therapeutically effective amount of that therapeutic agent.

As used herein, "pharmaceutically acceptable" refers to a substance that has been approved by the appropriate agency(ies) for use in animal species, again, in particular, human beings. This includes, of course, drugs but also includes other materials that, while not drugs *per se*, have a utility in animal species for other purposes. This includes substances that have not undergone extensive pharmacological testing but have been tested for safety and are found to be "generally regarded as safe" (GRAS) in animal species.

As used herein a "pharmaceutically acceptable antioxidant stabilizer" refers to a chemical substance that does not, at least in sufficiently low doses, detrimentally affect the physiological well-being of a patient to whom it has been administered and that is capable of preventing damage to therapeutic agents due to reaction of the agent with oxygen or free radicals released by reaction of oxygen with other entities. For the purpose of this invention, a pharmaceutically acceptable antioxidant includes, without limitation, a butylated phenol, butylated hydroxytoluene (BHT), butylated hydroxyanisole (BHA), t-butylhydroquinone, quinone, an alkyl gallate, methyl gallate, ethyl gallate, propyl gallate, octyl gallate, docecyl gallate, resveratrol, cysteine, n-acetylcysteine, bucillamine, glutathione, 7-hydroxyethylrutoside, carvedilol, vitamin C, ascorbyl palmitate, fumaric acid, a tocopherol, α-tocopherol, D,L-α-tocopherol, α-tocopherol acetate, a tocotrienol, vitamin E, lycopene, a flavonoid, a carotenoid and carotene. A presently preferred pharmaceutically acceptable antioxidant stabilizer for use in device and methods herein is butylated hydroxytoluene (BHT).

As noted previously, antioxidants of the type used for the stabilization of drugs herein are not particularly beneficial to patients. Significantly, this is not a great problem in theory. Once an implantable medical device has been fabricated and placed in protective packaging or once it has been implanted in a patient's body there is no further need of the antioxidant stabilizer. For example, a Xience V® (XV) stent (Abbott Vascular) in which the drug reservoir layer comprises everolimus as the macrocyclic triene lactone drug and BHT as the antioxidant stabilizer and which is packaged in a light-tight package under an Argon atmosphere, shelf life studies have shown that BHT is not necessary for product stability under long term (25 °C, 60% relative humidity) or intermediate (30°C, 65% relative humidity) storage conditions. Critical attributes of the coated device such as Total Content, Drug Release and Degradation Products have been found to be unaffected by the absence of BHT on the device. The preceding suggests that it would be beneficial to patients, and not detrimental to the macrocyclic triene lactone drug, to minimize the amount of antioxidant stabilizer on a finished, that is, fully fabricated, implantable medical device.

Thus, in an embodiment of this invention, a pharmaceutically acceptable antioxidant stabilizer may be included on an implantable medical device in an amount by weight that totals about 0.0001% to about 0.01 % of the total amount by weight of macrocyclic triene lactone present on the device.

To achieve the above amounts of residual antioxidant stabilizer on an implantable medical device, the relative free-radical sensitivity of the particular macrocyclic triene lactone drug and the efficacy of the selected antioxidant stabilizer in relation to the process by which the drug reservoir layer is coated on the device must be considered. The approach is empirical. That is, once a coating technique, a macrocyclic triene lactone drug and an antioxidant stabilizer have been selected, studies are performed to determine the effect of the manufacturing steps on the antioxidant stabilizer, the goal being to begin with sufficient antioxidant stabilizer in the coating solution to result in the desired quantity at the end of the fabrication process.

For example, with regard to the above-mentioned Xience V® stent, the process steps for coating the stent include spray coating a drug reservoir layer solution comprising everolimus and BHT onto a stent, which includes a primer layer, then drying the coated stent at an elevated temperature, mounting the stent on a delivery catheter and, finally, sterilizing the mounted stent/catheter. As seen in Fig.1, it was found that the amount of BHT present in the drug reservoir layer is reduce by about 61% during the spray coating step, by about 9% during the drying step, about 1% during the catheter mounting step and by about 64% during an ethylene oxide sterilization step. Fig. 1 relates to a stent comprising everolimus as the macrocyclic triene lactone and BHT as the antioxidant stabilizer but it is understood that similar if not identical results would be observed if a different macrocyclic triene lactone were used with BHT. Likewise it is expected that similar results would be obtained in terms of the magnitude of the effect at each step of a different antioxidant stabilizer were used. To confirm the large effect of sterilization on the BHT, a follow-up ethylene oxide sterilization was performed on the already sterilized catheter/stent and it was found that the BHT was reduced by an additional 30%.

Calculation of the proper amount of BHT to include with everolimus on a stent to arrive at a finished catheter/stent product that has the desired amount of residual BHT, i.e., about 0.001% to about 0.01% w/w based on the weight of everolimus on the stent, can be accomplished in two ways. At one extreme is stoichiometric reduction in the amount of BHT on the stent as the fabrication steps progress. In this approach, the amount of BHT drops by a constant amount at each fabrication step independent of the starting amount of BHT present. At the other extreme is a percentage drop where amount of the BHT decreases by the same percentage in each fabrication step, likewise independent of starting amount of BHT. Table 1 exemplifies these extremes compared to the known loss of BHT using the current Xience V® stent and everolimus, which contains 0.20% (w/w) of BHT based on the weight of everolimus.

**TABLE 1**

| | % Antioxidant (w/w, based on everolimus) as initially provided in everolimus for coating on XV stent | % Antioxidant (w/w, based on everolimus) in finished product |
|---|---|---|
| Current XV process | 0.20 | 0.044 |
| Assuming same proportional drop to achieve 0.01% residual | < 0.0455 | < 0.01 |
| Assuming same absolute amount of decrease to achieve 0.01% | < 0.166 | < 0.01 |

Thus, the fabrication process shown in Fig. 1 affords a drop in the amount of BHT from 0.2% in the starting material down to approximately 0.044% in the finished product. To achieve a final amount of BHT that is between the presently preferred 0.0001% and 0.01%, assuming the same proportional decrease in the amount of BHT, a starting amount in the coating solvent would be about 0.0455% (w/w) or less. On the other hand, assuming the same absolute decrease in the amount of BHT present in the finished product (stoichiometric loss), would require something less than about 0.166% (w/w) BHT in the coating solvent. Of course, a BHT reduction process that is neither stoichiometric nor proportional is possible, in which case the starting amount of BHT on the stent would be between the two extremes. It is clear, however, that beginning with about 0.0166% (w/w) BHT based on everolimus would in all instances result in a finished product have less that 0.01% (w/w) of BHT, an amount that would for all intents and purposes result in an insignificant exposure of a patient to the antioxidant.

The macrocyclic triene lactone drug may be essentially crystalline, essentially amorphous or anywhere in between. By "essentially crystalline" or "essentially amorphous" is meant that, while the bulk of a sample of the macrocyclic triene lactone drug will exhibit the characteristics of crystallinity or amorphousness, a small amount of the other particle form may still be detected in the sample. With regard specifically to everolimus, it is presently preferred that it be essentially amorphous, even more preferred is that it be amorphous within the detection limit of an appropriate method to detect the crystalline form as, for example, by differential scanning calorimetry.

As used herein, a "macrocyclic triene lactone" or "MTL" refers generally to a compound having a ring structure that contains 12 or more atoms, at least three conjugated double bonds and a lactone moiety in the ring system. In particular, "macrocyclic triene lactone" refers to rapamycin and derivatives thereof, including, at present, rapamycin itself, commonly known as sirolimus, 40-O-substituted rapamycins, 16-O-substituted rapamycins, rapamycin derivatives, 32-deoxorapamycin, zotarolimus, everolimus, temsirolimus, deforolimus, ridaforolimus, merilimus, biolimus, umirolimus, novolimus and myolimus. These compounds are "active agents" as set forth herein and are all mTOR inhibitors useful in the treatment of patients with damaged endothelia such as that which generally accompanies treatments such as PTCA, for vascular disease.

Presently preferred from among the macrocyclic triene lactones is everolimus.

The rapamycin macrocyclic triene lactones are oxygen sensitive due to the presence of the conjugated triene, i.e., three double bonds linked together by a single bond between the first and the second and a single bond between the second and the third. Since it is desirable, if not essential, that the composition and quantity of an active agent being administered to a patient, regardless of the manner of administration, be accurately known, it is highly desirable to control as well as possible any mechanism that might detrimentally affect the active agent before it is administered. Oxidation of compounds often has such a detrimental effect on active agents and is to be controlled. With regard to delivery of macrocyclic triene lactone active agents of this invention using implantable medical devices such as stents, a solution to this problem lies in the inclusion of pharmaceutically acceptable antioxidant compounds on the device. As noted previously "pharmaceutically acceptable" as used herein means that the antioxidants that are useful in this invention have been found acceptable for use in humans by the Food and Drug Administration (FDA, in the United States; equivalent foreign governmental agencies would be charged with such approvals in their respective countries). Of course, antioxidants that may in the future be found acceptable for human use by the FDA are clearly within the scope of this invention. Antioxidants curtail oxidation of macrocyclic triene lactones by several well-known mechanisms such as free radical scavenging and complexation with pro-oxidation metal species. BHT, a presently preferred antioxidant for use with an implantable medical device of this invention, is of the former type, i.e., it functions as a free radical scavenger.

If desired it is entirely possible, and in fact is an aspect of this invention, to include another therapeutic agent or agents along with the macrocyclic triene on an implantable medical device hereof. If the other agent(s) are known to not be oxygen sensitive, then no changes need be made to the disclosure herein of the amount of antioxidant to use. If, on the other hand, any of the additional therapeutic agents are known to be oxygen sensitive, then the total amount of antioxidant used may be determined as set forth above except that the total amount of macrocyclic triene lactone plus the amount of any other oxygen sensitive therapeutic agent(s) is used in the experiments performed to determine the effect of the fabrication steps on the amount of antioxidant stabilizer consumed during each phase of the fabrication.

Among other therapeutic agents that may be suitable for use in this invention, anti-inflammatory compounds are particularly presently preferred. Suitable anti-inflammatory agents that can be used in combination with the macrocyclic triene lactones herein include, without limitation, dexamethasone, dexamethasone acetate, clobetasol, alclofenac, alclometasone dipropionate, algestone acetonide, alpha amylase, amcinafal, amcinafide, amfenac sodium, amiprilose hydrochloride, anakinra, anirolac, anitrazafen, apazone, balsalazide disodium, bendazac, benoxaprofen, benzydamine hydrochloride, bromelains, broperamole, budesonide, carprofen, cicloprofen, cintazone, cliprofen, clobetasol propionate, clobetasone butyrate, clopirac, cloticasone propionate, cormethasone acetate, cortodoxone, deflazacort, desonide, desoximetasone, dexamethasone dipropionate, diclofenac potassium, diclofenac sodium, diflorasone diacetate, diflumidone sodium, diflunisal, difluprednate, diftalone, dimethyl sulfoxide, drocinonide, endrysone, enlimomab, enolicam sodium, epirizole, etodolac, etofenamate, felbinac, fenamole, fenbufen, fenclofenac, fenclorac, fendosal, fenpipalone, fentiazac, flazalone, fluazacort, flufenamic acid, flumizole, flunisolide acetate, flunixin, flunixin meglumine, fluocortin butyl, fluorometholone acetate, fluquazone, flurbiprofen, fluretofen, fluticasone propionate, furaprofen, furobufen, halcinonide, halobetasol propionate, halopredone acetate, ibufenac, ibuprofen, ibuprofen aluminum, ibuprofen piconol, ilonidap, indomethacin, indomethacin sodium, indoprofen, indoxole, intrazole, isoflupredone acetate, isoxepac, isoxicam, ketoprofen, lofemizole hydrochloride, lomoxicam, loteprednol etabonate, meclofenamate sodium, meclofenamic acid, meclorisone dibutyrate, mefenamic acid, mesalamine, meseclazone, methylprednisolone suleptanate, momiflumate, nabumetone, naproxen, naproxen sodium, naproxol, nimazone, olsalazine sodium, orgotein, orpanoxin, oxaprozin, oxyphenbutazone, paranyline hydrochloride, pentosan polysulfate sodium, phenbutazone sodium glycerate, pirfenidone, piroxicam, piroxicam cinnamate, piroxicam olamine, pirprofen, prednazate, prifelone, prodolic acid, proquazone, proxazole, proxazole citrate, rimexolone, romazarit, salcolex, salnacedin, salsalate, sanguinarium chloride, seclazone, sermetacin, sudoxicam, sulindac, suprofen, talmetacin, talniflumate, talosalate, tebufelone, tenidap, tenidap sodium, tenoxicam, tesicam, tesimide, tetrydamine, tiopinac, tixocortol pivalate, tolmetin, tolmetin sodium, triclonide, triflumidate, zidometacin, zomepirac sodium, aspirin (acetylsalicylic acid), salicylic acid, corticosteroids, glucocorticoids, tacrolimus, pimecorlimus and prodrugs, co-drugs and combinations thereof.

Other therapeutic agents that may be suitable for use in the methods herein include anti-neoplastic, antimitotic, antiplatelet, antifebrin, antithrombin, cytostatic and anti-proliferative agents.

Antineoplastic or anti-mitotic agents include, without limitation, paclitaxel, docetaxel, methotrexate, azathioprine, vincristine, vinblastine, fluorouracil, doxorubicin hydrochloride, and mitomycin.

Antiplatelet, anticoagulant, antifibrin, and antithrombin agents include, without limitation, sodium heparin, low molecular weight heparins, heparinoids, hirudin, argatroban, forskolin, vapiprost, prostacyclin, prostacyclin dextran, D-phe-pro-arg-chloromethylketone, dipyridamole, glycoprotein IIb/IIIa platelet membrane receptor antagonist antibody, recombinant hirudin and thrombin, thrombin inhibitors such as Angiomax ä, calcium channel blockers such as nifedipine, colchicine, fish oil (omega 3-fatty acid), histamine antagonists, lovastatin, monoclonal antibodies (such as those specific for Platelet-Derived Growth Factor (PDGF) receptors), nitroprusside, phosphodiesterase inhibitors, prostaglandin inhibitors, suramin, serotonin blockers, steroids, thioprotease inhibitors, triazolopyrimidine (a PDGF antagonist), nitric oxide or nitric oxide donors, super oxide dismutases, super oxide dismutase mimetic, 4-amino-2,2,6,6-tetramethylpiperidine-1-oxyl (4-amino-TEMPO) and estradiol.

Cytostatic or anti-proliferative agents include, without limitation, angiopeptin, angiotensin converting enzyme inhibitors such as captopril, cilazapril or lisinopril, calcium channel blockers such as nifedipine; colchicine, fibroblast growth factor (FGF) antagonists; fish oil (ω-3-fatty acid); histamine antagonists; lovastatin, monoclonal antibodies such as, without limitation, those specific for Platelet-Derived Growth Factor (PDGF) receptors; nitroprusside, phosphodiesterase inhibitors, prostaglandin inhibitors, suramin, serotonin blockers, steroids, thioprotease inhibitors, triazolopyrimidine (a PDGF antagonist) and nitric oxide.

Other potentially useful therapeutic agents include, without limitation, alpha-interferon, genetically engineered epithelial cells, DNA and RNA nucleic acid sequences, antisense molecules, and ribozymes, antibodies, receptor ligands, enzymes, adhesion peptides, blood clotting factors, inhibitors or clot dissolving agents such as streptokinase and tissue plasminogen activator, antigens for immunization, hormones and growth factors, oligonucleotides, retroviral vectors; antiviral agents; analgesics; anorexics; antihelmintics; antiarthritics, antiasthmatic agents; anticonvulsants; antidepressants; antidiuretic agents; antidiarrheals; antihistamines; antimigrain preparations; antinauseants; antiparkinsonism drugs; antipruritics; antipsychotics; antipyretics; antispasmodics; anticholinergics; sympathomimetics; xanthine derivatives; cardiovascular preparations including calcium channel blockers, beta-blockers such as pindolol, antiarrhythmics; antihypertensives; diuretics; vasodilators including general coronary; peripheral and cerebral; central nervous system stimulants; cough and cold preparations, including decongestants; hypnotics; immunosuppressives; muscle relaxants; parasympatholytics; psychostimulants; sedatives; tranquilizers; natural or genetically engineered lipoproteins; and restenosis reducing agents.

The drug reservoir layer of this invention may be prepared and applied to an implantable medical device in the following manner: an implantable medical device is first provided. By "provided" is meant that the device is provided to the process being discussed, not to the person carrying out the process. That is, the operator "provides" all the elements, the device, the drug, the antioxidant, etc. that is needed to perform the process. The term is not intended nor should it be construed as involving any third party not involved in the actual performance of the claimed process.

A macrocyclic triene lactone and pharmaceutically acceptable antioxidant stabilizer are next provided under the same proviso set forth above; i.e., "provided" to the process by the persons performing same and the two are combined. The macrocyclic triene lactone drug and antioxidant stabilizer may be combined in the dry state. "Combining" the drug and the antioxidant may be accomplished by simply mixing the substances together. Alternatively, the macrocyclic triene lactone and antioxidant may be dissolved in a water miscible solvent and then the two compounds may be co-precipitated by adding the solution to an excess of water, which results in the macrocyclic triene lactone and antioxidant being intimately mixed at the molecular level. The co-precipitate may then be dried and then dissolved or dispersed in a coating solvent or, if the selected coating solvent is water miscible, the co-precipitant may be used as is, still wetted with water. Alternately, if supplied separately, the drug and antioxidant may be sequentially added to a coating solvent. Polymers for controlling the drug release and providing for a means to secure the drug to the stent may be added and dissolved or dispersed in a coating solvent. A coating solvent simply refers to a liquid that is capable of dissolving one or both compounds or simply acting as a carrier for the compounds in a dispersed state and that is sufficiently volatile to permit drying of the coated layer under relatively mild conditions and at a temperature that does not detrimentally affect either the macrocyclic triene lactone drug or the antioxidant stabilize. A host of suitable coating solvents are well-known to those skilled in the art. Any of the known solvents and any that become known in the future will be suitable for use in the method of this invention and are all within the scope of this invention. On the other hand, combining the substances may comprise individually or collectively, i.e., after mixing the compounds together, mechanically as by, without limitation, physical grinding reducing the particle size of both substances to micro- or nano-scale particles. By "micro-scale" is meant an average particle size of about 0.1micrometer to about 100 micrometers. By "nano-scale" is meant an average particle size of about 1 nanometer to about 100 nanometers. If ground separately, the resulting powders are then mixed together and then dissolved or dispersed in a coating solvent. If the antioxidant stabilizer is BHT, combining the drug and the BHT may also comprise reducing the particle size of the macrocyclic triene lactone drug to micro- or nano-scale particles, mixing the resultant powder with BHT, placing the mixture in a vessel capable of accommodating a reduced pressure, reducing the pressure in the vessel to a pressure at which BHT will sublime at a temperature that is determined to not detrimentally affect the drug and heating the vessel to the proper temperature while continuously agitating the mixture. Once it has been determined that all of the BHT has sublimed and deposited on the drug, the temperature is reduced to ambient, the vacuum is released and the resultant powder is dissolved or dispersed in a coating solvent.

Regardless of how the macrocyclic triene lactone and the antioxidant stabilizer are combined, the amount of antioxidant stabilizer is about 0.02 % (w/w) to about 0.1% (w/w) based on the amount of macrocyclic triene lactone.

The dissolved or dispersed powder is then spray-coated on the implantable medical device to form a drug reservoir layer in any of several manners well-known to those skilled in the art. Spray coating may comprise a one-pass coating or a plurality of passes to achieve the desired coating thickness and quantity of drug on the implantable device.

The spray-coated implantable device is then dried at an elevated temperature, which as above, is selected so as to have no detrimental effect on the macrocyclic triene lactone drug, a temperature that is empirically determined by experimentation with the drug.

The implantable medical device comprising at least the drug reservoir layer is then mounted on a delivery vehicle, packaged and the mounted implantable device is sterilized. In the case of a DES, the stent is usually mounted on a catheter as the delivery device.

Sterilization of the mounted device by ethylene oxide sterilization, e-beam sterilization or gamma sterilization, are well-known to those skilled in the art and require no detailed description. If ethylene oxide sterilization is selected, the mounted implantable medical device may be packaged in a gas permeable container and then subjected to the sterilization procedure. Subsequent to ethylene oxide sterilization, the gas permeable container is further packaged in a light-tight container under an inert atmosphere such as, without limitation, argon gas.

The sterilized device/carrier vehicle is then ready for use or for storage.

It is a presently preferred embodiment for purposes of this invention that the implantable medical device is a stent, the macrocyclic triene lactone drug is everolimus, the antioxidant stabilizer is BHT and the delivery vehicle is a catheter.

## Claims

1. A method of fabricating an implantable medical device comprising a drug reservoir layer comprising a macrocyclic triene lactone drug and less than 0.01% by weight, based on the weight of macrocyclic triene lactone present, of a pharmaceutically acceptable antioxidant stabilizer, the method comprising,
providing an implantable medical device wherein:
the implantable medical device may be a device body or it may be a device body that has already been coated with one or more layers of material(s);
providing an essentially pure macrocyclic triene lactone drug;
providing a pharmaceutically acceptable antioxidant stabilizer in an amount of 0.02% to 0.1% by weight based on the weight of the macrocyclic triene lactone drug to be disposed on the device body;
combining the macrocyclic triene lactone drug and pharmaceutically acceptable antioxidant stabilizer;
dissolving or dispersing the combined macrocyclic triene lactone drug and pharmaceutically acceptable antioxidant stabilizer in a coating solvent;
spray-coating the drug/stabilizer-containing solvent onto the implantable medical device body;
drying the spray-coated implantable medical device at an elevated temperature that has been determined to not detrimentally affect the macrocyclic triene lactone drug;
mounting the dried spray-coated implantable medical device on a carrier vehicle; and
sterilizing the mounted implantable medical device/carrier vehicle;
wherein subsequent to mounting and prior to sterilization, the mounted implantable medical device/carrier vehicle is packaged in a gas permeable container; and
wherein sterilization comprises ethylene oxide.

2. The method of claim 1, wherein a matrix polymer is added to the macrocyclic triene lactone drug and antioxidant stabilizer in the dissolution or dispersion step.

3. The method of claim 1, wherein the sterilized implantable medical device/carrier vehicle/gas permeable container is further packaged in a light-tight container under an inert atmosphere.

4. The method of claim 1, wherein combining the macrocyclic triene lactone drug with the pharmaceutically acceptable antioxidant stabilizer comprises mechanically reducing the particle size of each substance in the solid state to form micro-scale or nano-scale powders, either separately, after which the powders are mixed together to form a substantially homogeneous mixed powder or mixing the solid substances together before mechanically reducing particle size.

5. The method of claim 1, wherein combining the macrocyclic triene lactone drug with the pharmaceutically acceptable antioxidant stabilizer comprises dissolving both in a water miscible solvent and then adding the solution to a volume of water to co-precipitate the macrocyclic triene lactone drug and antioxidant stabilizer, the co-precipitant then being used in the dissolution/dispersion step.

6. The method of claim 1, wherein the implantable medical device is a stent.

7. The method of claim 1, wherein the macrocyclic triene lactone drug is selected from the group consisting of rapamycin, a 40-O-substituted rapamycin, a 16-O-substituted rapamycin, a rapamycin derivative, 32-deoxorapamycin, zotarolimus, everolimus, temsirolimus, deforolimus, ridaforolimus, merilimus, biolimus, umirolimus, novolimus and myolimus.

8. The method of claim 1, wherein the pharmaceutically acceptable antioxidant stabilizer is selected from the group consisting of a butylated phenol, butylated hydroxytoluene (BHT), butylated hydroxyanisole, t-butylhydroquinone, quinone, an alkyl gallate, methyl gallate, ethyl gallate, propyl gallate, octyl gallate, docecyl gallate, resveratrol, cysteine, n-acetylcysteine, bucillamine, glutathione, 7-hydroxyethylrutoside, carvedilol, vitamin C, ascorbyl palmitate, fumaric acid, a tocopherol, α-tocopherol, D,L-α-tocopherol, α-tocopherol acetate, a tocotrienol, vitamin E, lycopene, a flavonoid, a carotenoid and carotene.

9. The method of claim 8, wherein the pharmaceutically acceptable antioxidant stabilizer is BHT.

10. The method of claim 9, wherein combining the macrocyclic triene lactone drug with the BHT comprises mixing the macrocyclic triene lactone drug solid, which has been ground to a micro- or nano-scale powder, and the BHT in a vessel under reduced pressure and heating the mixture to a temperature above the sublimation temperature of the BHT at that reduced pressure.

11. The method of claim 10, wherein the macrocyclic triene lactone drug is selected from the group consisting of rapamycin, novolimus, everolimus and zotarolimus.

## Patentansprüche

1. Ein Verfahren zur Herstellung von einem implantierbaren medizinischen Gerät umfassend eine Arzneimittelreservoirschicht umfassend ein makrozyklisches Trienlactonarzneimittel und weniger als 0,01 Gew.-%, bezogen auf das Gewicht des vorhandenen makrozyklischen Trienlactons, von einem pharmazeutisch akzeptablen Antioxidationsstabilisator, wobei das Verfahren folgendes umfasst,
bereitstellen von einem implantierbaren medizinischen Gerät, wobei:
das implantierbare medizinische Gerät ein Gerätekörper sein kann oder ein Gerätekörper sein kann, der bereits mit einer oder mehreren Schichten von einem oder mehreren Stoff(en) beschichtet worden ist;
bereitstellen von einem im Wesentlichen reinen makrozyklischen Trienlactonarzneimittel;
bereitstellen von einem pharmazeutisch akzeptablen Antioxidationsstabilisator in einer Menge von 0,02 Gew.-% bis 0,1 Gew.-% bezogen auf das Gewicht des auf den Gerätekörper anzuordnenden makrozyklischen Trienlactonarzneimittels;
kombinieren des makrozyklischen Trienlactonarzneimittels und des pharmazeutisch akzeptablen Antioxidationsstabilisators;
lösen oder dispergieren des kombinierten makrozyklischen Trienlactonarzneimittels und pharmazeutisch akzeptablen Antioxidationsstabilisators in einem Beschichtungslösungsmittel;
sprühbeschichten von dem Arzneimittel/stabilisatorenthaltenden Lösungsmittel auf den Körper des implantierbaren medizinischen Geräts;
trocknen des sprühbeschichteten implantierbaren medizinischen Geräts bei einer erhöhten Temperatur, die als nicht schädlich für das makrozyklische Trienlactonarzneimittel festgestellt worden ist;
montieren des getrockneten sprühbeschichteten implantierbaren medizinischen Geräts auf einen Trägerstoff; und
sterilisieren von dem montierten implantierbaren medizinischen Gerät/auf Trägerstoff;
wobei nach der Montage und vor der Sterilisation, das montierte implantierbare medizinische Gerät/auf Trägerstoff in einem gasdurchlässigen Gefäß verpackt wird; und
wobei die Sterilisation Ethylenoxid umfasst.

2. Das Verfahren des Anspruchs 1, wobei ein Matrixpolymer dem makrozyklischen Trienlactonarzneimittel und Antioxidationsstabilisator im Lösungs- oder Dispersionsschritt hinzugegeben wird.

3. Das Verfahren des Anspruchs 1, wobei das sterilisierte implantierbare medizinische Gerät/auf Trägerstoff/im gasdurchlässigen Gefäß weiterhin in einem lichtdichten Gefäß unter einer inerten Atmosphäre verpackt wird.

4. Das Verfahren des Anspruchs 1, wobei das Kombinieren des makrozyklischen Trienlactonarzneimittels mit dem pharmazeutisch akzeptablen Antioxidationsstabilisator folgendes umfasst: das mechanische Reduzieren der Partikelgröße von jeder Substanz im festen Zustand, um mikroskalige oder nanoskalige Pulver zu bilden, entweder getrennt voneinander, nach dem die Pulver zusammen gemischt werden, um ein im Wesentlichen homogenes gemischtes Pulver zu bilden, oder das Mischen der festen Substanzen zusammen vor der mechanischen Reduktion der Partikelgröße.

5. Das Verfahren des Anspruchs 1, wobei das Kombinieren des makrozyklischen Trienlactonarzneimittels mit dem pharmazeutisch akzeptablen Antioxidationsstabilisator folgendes umfasst: das Lösen von den beiden in einem wassermischbaren Lösungsmittel und das darauf folgende Hinzugeben der Lösung einem Wasservolum, damit das makrozyklische Trienlactonarzneimittel und der Antioxidationsstabilisator co-präzipitieren, wobei der Co-Präzipitant danach im Lösung-/Dispersionsschritt verwendet wird.

6. Das Verfahren des Anspruchs 1, wobei das implantierbare medizinische Gerät ein Stent ist.

7. Das Verfahren des Anspruchs 1, wobei das makrozyklische Trienlactonarzneimittel ausgewählt ist aus der Gruppe bestehend aus Rapamycin, einem 40-O-substituierten Rapamycin, einem 16-O-substituierten Rapamycin, einem Rapamycinderivat, 32-Deoxorapamycin, Zotarolimus, Everolimus, Temsirolimus, Deforolimus, Ridaforolimus, Merilimus, Biolimus, Umirolimus, Novolimus und Myolimus.

8. Das Verfahren des Anspruchs 1, wobei der pharmazeutisch akzeptable Antioxidationsstabilisator ausgewählt ist aus der Gruppe bestehend aus einem butylierten Phenol, butylierten Hydroxytoluol (BHT), butylierten Hydroxyanisol, *tert-*Butylhydrochinon, Chinon, einem Alkylgallat, Methylgallat, Ethylgallat, Propylgallat, Octylgallat, Docecylgallat, Resveratrol, Cystein, n-Acetylcystein, Bucillamin, Glutathion, 7-Hydroxyethylrutosid, Carvedilol, Vitamin C, Ascorbylpalmitat, Fumarsäure, einem Tocopherol, α-Tocopherol, D,L-α-Tocopherol, α-Tocopherolacetat, einem Tocotrienol, Vitamin E, Lycopen, einem Flavonoid, einem Carotenoid und Caroten.

9. Das Verfahren des Anspruchs 8, wobei der pharmazeutisch akzeptable Antioxidationsstabilisator BHT ist.

10. Das Verfahren des Anspruchs 9, wobei das Kombinieren des makrozyklischen Trienlactonarzneimittels mit dem BHT das Mischen des makrozyklischen Trienlactonarzneimittelfeststoffs, welcher zu einem mikroskaligen oder nanoskaligen Pulver gemahlen worden ist, und des BHT in einem Behälter unter reduziertem Druck und das Erhitzen der Mischung auf eine Temperatur oberhalb der Sublimationstemperatur des BHT bei dem reduzierten Druck umfasst.

11. Das Verfahren des Anspruchs 10, wobei das makrozyklische Trienlactonarzneimittel ausgewählt aus der Gruppe bestehend aus Rapamycin, Novolimus, Everolimus und Zotarolimus ist.

## Revendications

1. Un procédé de fabrication d'un dispositif médical implantable comprenant une couche de réservoir de médicament comprenant un médicament de triènelactone macrocyclique et moins de 0,01 % en poids, par rapport au poids de triènelactone macrocyclique présente, d'un stabilisateur antioxydant pharmaceutiquement acceptable, le procédé comprenant,
fournir un dispositif médical implantable dans lequel :
le dispositif médical implantable peut être un corps de dispositif ou il peut être un corps de dispositif qui a déjà été recouvert avec une ou plusieurs couches de matériau(x) ;
fournir un médicament de triènelactone macrocyclique essentiellement pur ;
fournir un stabilisateur antioxydant pharmaceutiquement acceptable dans une quantité de 0,02 % à 0,1 % en poids par rapport au poids du médicament de triènelactone macrocyclique devant être disposé sur le corps de dispositif ;
combiner le médicament de triènelactone macrocyclique et le stabilisateur antioxydant pharmaceutiquement acceptable ;
dissoudre ou disperser le médicament de triènelactone macrocyclique et le stabilisateur antioxydant pharmaceutiquement acceptable combinés dans un solvant de revêtement ;
appliquer par pulvérisation un revêtement du médicament/solvant contenant du stabilisateur sur le corps de dispositif médical implantable ;
sécher le dispositif médical implantable revêtu par pulvérisation à une température élevée qui a été déterminée comme n'ayant pas d'effet négatif sur le médicament de triènelactone macrocyclique ;
monter le dispositif médical implantable revêtu par pulvérisation séché sur un véhicule porteur ; et
stériliser le dispositif médical implantable monté/véhicule porteur ;
dans lequel après le montage et avant la stérilisation, le dispositif médical implantable monté/véhicule porteur est emballé dans un récipient perméable aux gaz ; et
dans lequel la stérilisation comprend de l'oxyde d'éthylène.

2. Le procédé de la revendication 1, dans lequel un polymère de matrice est ajouté au médicament de triènelactone macrocyclique et au stabilisateur antioxydant dans l'étape de dissolution ou de dispersion.

3. Le procédé de la revendication 1, dans lequel le dispositif médical implantable stérilisé/véhicule porteur/récipient perméable aux gaz est emballé en outre dans un récipient étanche à la lumière sous une atmosphère inerte.

4. Le procédé de la revendication 1, dans lequel combiner le médicament de triènelactone macrocyclique avec le stabilisateur antioxydant pharmaceutiquement acceptable comprend réduire mécaniquement la taille des particules de chaque substance dans l'état solide afin de former des poudres à l'échelle micro ou à l'échelle nano, bien séparément, après lequel les poudres sont mélangées les unes avec les autres afin de former une poudre mélangée essentiellement homogène ou bien mélanger les substances solides conjointement avant de réduire mécaniquement la taille des particules.

5. Le procédé de la revendication 1, dans lequel combiner le médicament de triènelactone macrocyclique avec le stabilisateur antioxydant pharmaceutiquement acceptable comprend dissoudre tous deux dans un solvant miscible en eau et ajouter alors la solution à un volume d'eau afin de co-précipiter le médicament de triènelactone macrocyclique et le stabilisateur antioxydant, le co-précipitant étant utilisé alors dans l'étape de dissolution/dispersion.

6. Le procédé de la revendication 1, dans lequel le dispositif médical implantable est un stent.

7. Le procédé de la revendication 1, dans lequel le médicament de triènelactone macrocyclique est choisi dans le groupe constitué de la rapamycine, une rapamycine à substitution 40-O, une rapamycine à substitution 16-O, un dérivé de rapamycine, la 32-déoxorapamycine, le zotarolimus, l'évérolimus, le temsirolimus, le déforolimus, le ridaforolimus, le mérilimus, le biolimus, l'umirolimus, le novolimus et le myolimus.

8. Le procédé de la revendication 1, dans lequel le stabilisateur antioxydant pharmaceutiquement acceptable est choisi dans le groupe constitué d'un phénol butylé, l'hydroxytoluène butylé (BHT), l'hydroxanisole butylé, la *tert-*butylhydroquinone, la quinone, un gallate d'alkyle, un gallate de méthyle, un gallate d'éthyle, un gallate de propyle, un gallate d'octyle, un gallate de docécyle, le resvératrol, la cystéine, la n-acétylcystéine, la bucillamine, le glutathion, le 7-hydroxyéthylrutoside, le carvédilol, la vitamine C, le palmitate d'ascorbyle, l'acide fumarique, un tocophérol, le α-tocophérol, le D,L-α-tocophérol, l'acétate de α-tocophérol, un tocotriénol, la vitamine E, le lycopène, un flavonoïde, un caroténoïde et le carotène.

9. Le procédé de la revendication 8, dans lequel le stabilisateur antioxydant pharmaceutiquement acceptable est le BHT.

10. Le procédé de la revendication 9, dans lequel combiner le médicament de triènelactone macrocyclique avec le BHT comprend mélanger le solide de médicament de triènelactone macrocyclique, qui a été moulu jusqu'à obtenir une poudre à échelle micro ou à échelle nano, et le BHT dans un réservoir sous pression réduite et échauffer le mélange jusqu'à une température au-dessus de la température de sublimation du BTH à ladite pression réduite.

11. Le procédé de la revendication 10, dans lequel le médicament de triènelactone macrocyclique est choisi dans le groupe constitué de la rapamycine, le novolimus, l'évérolimus et le zotarolimus.
